# EUROPEAN PATENT SPECIFICATION

(11) **EP 3 052 091 B1**
(45) Date of publication and mention of the grant of the patent: **24.04.2019**
(21) Application number: 13792770.3
(22) Date of filing: 01.10.2013
(51) Int. Cl.: A61K 9/51, A61K 31/121, A61P 35/00, A61P 31/10, A61P 37/02

(54) **SUSTAINED RELEASE FORMULATIONS CONTAINING METHYLGLYOXAL AND THEIR THERAPEUTIC APPLICATIONS**
VERZÖGERT FREIGESETZTE FORMULIERUNGEN MIT METHYLGLYOXAL UND DEREN THERAPEUTISCHEN ANWENDUNGEN
FORMULATIONS À LIBÉRATION PROLONGÉE CONTENANT DU MÉTHYLGLYOXAL ET LEUR APPLICATIONS THÉRAPEUTIQUES

(43) Date of publication of application: 10.08.2016
(73) Proprietor: Lifecare Innovations Pvt. Ltd., Kolkata 700031 (IN)
(72) Inventor: MANJU, Ray, Kolkata 700092 (IN); JINTEDRA, Nath Verma, Gurgaon 122002 (IN); LILY, Verma, Gurgaon 122002 (IN)
(74) Representative: Zimmermann, Tankred Klaus
(86) International application number: PCT/IN2013/000598
(87) International publication number: WO 2015/049689

(56) References cited:
- US-A1- 2011 118 327
- Manas P. Gupte: "Preparation and Characterization of Methylglyoxal Nanoparticulate Drug Delivery System", Thesis Creighton University , 14 September 2012 (2012-09-14), pages 1-99, XP002724633, Omaha, NE Retrieved from the Internet: URL:https://dspace.creighton.edu/xmlui/bit stream/handle/10504/23037/M_Gupte-thesis.p df?sequence=1 [retrieved on 2014-05-19]
- Anonymous: "Preparation and Characterization of Methylglyoxal Nanoparticulate Drug Delivery System. DSpace/Manakin Repository", Creighton University , pages 1-6, XP002724634, Retrieved from the Internet: URL:https://dspace.creighton.edu/xmlui/han dle/10504/23037?show=full [retrieved on 2014-05-19]
- ADRITA CHAKRABARTI ET AL: "Immunomodulation of macrophages by methylglyoxal conjugated with chitosan nanoparticles against Sarcoma-180 tumor in mice", CELLULAR IMMUNOLOGY, vol. 287, no. 1, 8 December 2013 (2013-12-08), pages 27-35, XP055118681, ISSN: 0008-8749, DOI: 10.1016/j.cellimm.2013.11.006

## Description

### FIELD OF INVENTION

The present invention relates to a sustained release formulation containing methylglyoxal for use in inhibition and/or treatment of malignancies with and without metastasis; for use in treating infections including fungal infections and as immune-modulator medicament, comprising of following constituents: 0.125-0.5 mg of methylglyoxal encapsulated by or conjugated to nanoparticles of bio-degradable synthetic polymers or natural polymers; 0.125-0.5 mg of melatonin; 75-300 mg of creatine; and 25-100 mg of ascorbic acid, wherein all doses are per kg of body weight. The present invention also relates to a process for the preparation of a nanoparticle drug formulation comprising: preparing a homogenous solution of chitosan; adding methylglyoxal solution to the homogenous solution to form a reaction mixture; adding a surfactant to the reaction mixture; subjecting the reaction mixture and surfactant to the step of stirring/mixing; adding sodium sulfate to the surfactant and reaction mixture under stirring/mixing; adding a crosslinking agent and finally sodium metabisulphite to the solution, subjecting the solution to the step of filtration.

### BACKGROUND OF THE INVENTION

Manas P. Gupte, "Preparation and Characterization of Methylglyoxal Nanoparticulate Drug Delivery System", Omaha, NE, (20120914), pages 1 - 99, Thesis Creighton University, URL: https://dspace.creighton.edu/xmlui/bitstream/handle/10504/23037/M_Guptethesis.pdf?sequence=1, discloses chitosan nanoparticles comprising methylglyoxal for the treatment of cancer. The dosage of methylglyoxal is not disclosed, and melatonin, creatine and ascorbic acid are also not disclosed. The nanoparticles are prepared by a method of using a cross linking agent and a surfactant, but the use of sodium sulfate and sodium metabisulfate is not disclosed.

According to a WHO report, Cancer is the third leading cause of death worldwide and no longer predominantly afflictions of wealthy countries. Cancer accounted for 7.6million deaths (around 13% of all deaths in 2008). Deaths due to Cancer worldwide are projected to continue rising with an estimated 13.1 million deaths in 2030. Worldwide, the percentage of all cases that occur in low- and middle-income countries has increased from 51 % in 1975 to 70% in 2008.

Early observations of Percival Pott, a British Surgeon, reported in 1775, made correlation between environmental agents (chemical carcinogen) as cause of cancer. Subsequently, over the last two centuries, several other causes of cancer were reported viz. ionizing radiation, UV radiation, radon gas, infections, immune system dysfunction, genetic mutation etc. Cancers are presently treated by surgical interventions, bone-marrow/stem cell transplantation, chemotherapy, radiotherapy, hormonal therapy, immunotherapy, monoclonal antibody therapy, or other methods including combinations of the outlined methods. Treatment options continue to evolve and in the last half a century commendable progress has been made. Yet, the limited options are far from satisfactory. The treatment options depend upon, type, site, stage, severity, age and general health of the patient. Any modality of therapy of cancer while killing the cancerous tissues/cells, involves possibility of risk of adversely affecting healthy tissues resulting in collateral toxicities and complications.

Immunotherapy, of all the modalities apparently is quite promising as body's self-defense system is complimented and encouraged to destroy cancerous tissues in a more specific manner. Such treatments, however, are not effective for all types of cancers. An ideal anticancer drug should be targeting only the cancerous cells without harming healthy tissues. None of the anticancer drugs being used presently, act specifically against cancerous cells and range from being moderate to highly toxic. In addition to toxicity, another important concern is regression devoid of complete cure resulting in metastasis and/or recurrence in most cases.

Despite extensive work to understand the causes of cancer and develop effective anti-cancer drugs and other treatment modalities, it is still not clear what regulates the invasive and metastatic properties of malignant cells. Number of research groups globally, are engaged into working on molecular biology to understand the differences between normal and malignant cells with an objective to delineate causes, pathogenesis and mechanism of therapeutic actions that would allow development of a truly target specific drug for cancer treatment.

With an objective of development of a target specific anticancer drug, our group has been engaged since over three decades, in research on molecular basis of differential action on healthy and malignant cells of an endogenous Ketoaldehyde - Methylglyoxal. It has been well known for long, that ketoaldehydes particularly, Methylglyoxal - an intermediate of glucose breakdown possesses anticancer properties. Methylglyoxal is a chemically and biochemically active, small molecule. Owing to a reactive aldehyde group and a ketonic group, it is an acceptor of electrons. In biological chemistry domain, this compound has a chequered history.

Neuberg and Kerb in 1913 proposed a metabolic pathway of alcoholic fermentation from glucose involving methylglyoxal as an intermediate. The anti-cancer properties of ketoaldehydes and their derivatives have been known since as early as 1958. Szent-Györgyi and his collaborators proposed that Methylglyoxal - a natural growth regulator along with Glyoxalase enzyme system, Methylglyoxal exerts control over cellular growth. The strong anti-cancer effect of Methylglyoxal demonstrated in animal systems has been ascribed to its distinct properties of malignant cell specific growth inhibition. No tumor developed and the mice remained completely healthy when sarcoma-180 cells were injected concomitantly with Methylglyoxal. Apple and Greenberg have also shown that a wide variety of cancers in mice could be treated with Methylglyoxal injections. Similar observations about the anti-cancer effects of Methylglyoxal have been reported by several other research groups. The reported findings suggested that the anticancer effect of Methylglyoxal is mediated through inhibition of protein synthesis and interaction with Nucleic Acids culminating into malignant cell growth inhibition. These works were done mainly with animal model systems and very little work was carried out with human tissue material. However, in 1970s and 1980s, the metabolic pathway for Methylglyoxal has been elucidated in different organisms including yeast, bacteria and protozoa with work involving the isolation, purification and characterization of several enzymes responsible for the formation and breakdown of Methylglyoxal. In addition to its antitumor effect, Methylglyoxal has also exhibited antiviral and antimalarial activity Interestingly, *Pseudomonas aeroginosa* resistant to Piperacillin becomes sensitive to the same antibiotic when administered along with Methylglyoxal.

Methylglyoxal has been dogmatically shown to act specifically against malignant cells and has tumouricidal effect. Further, it has also been reported that as compared to healthy cells, cancer cells are more sensitive to methylglyoxal. Recently, it has been shown that Methylglyoxal inhibits both Glyceraldehyde-3-Phospahte Dehydrogenase and Mitochondrial Complex I of specifically malignant cells, while Methylglyoxal has no inhibitory effect on these enzymes of normal cells. These observations suggest the possibility of alternation of these two enzymes in malignant cells. 85% of the ATP in cells is generated by the catalytic activities of these two enzymes and thus the inhibitory action of these enzymes of malignant cells depletes these cells of their ATP pool rendering the cells non-viable. The kinetics and structure of Glyceraldehydes-3-Phosphate Dehydrogenase that catalyzes the phosphorylation of D-Glyceraldehyde-3-Phosphate to 1,3 Biphosphoglycerate enzyme may be critically altered in malignant cells. Glyceraldehyde-3-Phosphate Dehydrogenase expression is altered in hypoxia, in oncogene transformed cells, and varies with the cell cycle. Several reports have established the role of Glyceraldehydes-3-Phosphate Dehydrogenase in malignancies viz, in lung cancers, pancreatic cancers, breast cancers and prostate cancers etc.

Methylglyoxal has been reported to induce apoptosis in leukemic cell lines and in human prostate carcinoma cells. In addition to anti-tumor activity, our group has also observed and reported immuno-modulatory role of Methylglyoxal. Cancer is an immuno-suppressive disease and Methylglyoxal strongly stimulates host's immune response against tumor cell by producing Reactive Oxygen Intermediates and Reactive Nitrogen Intermediates which leads to enhancement of macrophages and lymphocyte activation or immuno-modulation against sarcoma-180 tumor.

In addition to *in vitro* studies on cell lines, the anticancer effect of Methylglyoxal had also been tested in animals establishing that Methylglyoxal has significant curative effect on wide variety of cancers. No tumor developed and the mice remained completely healthy when experimental mice inoculated with ascites Sarcoma 180 cells were also given Methylglyoxal. Inhibition of the Ehrlich Ascites Carcinoma (EAC) cells and Leukemia cells proliferation by Methylglyoxal has also been demonstrated. Subsequent *in vitro* and *in vivo* studies from different laboratories had corroborated this remarkable anticancer effect of Methylglyoxal. Recently Ray *et al* have observed that Methylglyoxal inhibits Sarcoma-180 cells induced Solid Tumor by blocking Angiogenesis.

Highly encouraging results were observed when Methylglyoxal was used as an anticancer drug in the form of aqueous solution along with Vitamin C, Vitamin B Complex, and some other Antioxidants. Methylglyoxal, however, is readily decomposed in the body by enzymatic degradation. Many investigators attempted to block the metabolic degradation of methylglyoxal so that the drug is retained in the body for longer duration enhancing therapeutic activity. These blocking agents however are mostly toxic in nature. Rapid bio-elimination of Methylglyoxal compels frequent and large dosing which can be improved by replacement with the sustained release formulation.

Versatilities of NDDS based controlled release formulations offer possibilities of targeted delivery at the malignant site and sustained release of the encapsulated drug over long duration thus enabling reduction dose and their frequency.

Amongst various carrier systems that have been employed and tested for drug delivery include liposomes, polymeric miscelles, dendrimers, polymer-drug conjugates polymeric nanoparticles and nanoparticles. Taking into account the objective of treating cancers with hydrophilic small molecule, hydrogel nanoparticles made of polymers appear promising to satisfy needs of strategically designed formulation as nano-drug carries with hydrophilic surfaces. These nanoparticles are known to evade recognition and uptake by the reticulo-endothelial systems (RES) and resultantly can circulate in the blood for long durations. Additionally, owing to their nano-size, these hydrophilic particles extravasate at the pathological sites such as solid tumors through passive targeting mechanism.

A hydrogel nanoparticle is usually constituted assembly of several hundreds of water soluble low molecular weight polymer and has a diameter of less than 100 nm. Both, the core, as well as shell of these nanoparticles being hydrophilic in nature, water-soluble drugs stay in the core whereas hydrophilic outer surface helps evade body's RES which permits circulation in the blood for a longer period of time. Since the surface of these hydrogel nanoparticles are chemically reactive, drugs and other active molecules can also be conjugated on the particle surface.

Extensive research has been done to examine the possibilities of employing polymeric nanoparticles as carriers for drug delivery. Variety of natural and synthetic biodegradable polymers such as Chitosan, Alginate, PLA, PLG, Poly-Epsilon-Caprolactone and lipids have been found to be suitable as for encapsulation and use as drug carrier.

Despite the knowledge that small drug molecules encapsulated into the hydrogel nanoparticles rapidly leak out of the particles through diffusion, this technique is promising as the nanoparticles are prepared under mild conditions without using harmful solvents. As organic solvents may cause degradation of many drugs that are insoluble, unstable and sensitive to their environments, alternative formulation processes avoiding these solvents are preferred.

The method has improvement over earlier reported methods. Unlike earlier methods, it is industrially adoptable and yields Methylglyoxal nano particles that have been designed and found to be more effective and safe.

Chitosan, is a cationic polysaccharide and it is generally prepared by alkaline deacetylation of Chitin. Chitosan is one such hydrogel material which is suitable for entrapment in as well as surface conjugation of water soluble chemical chemical compounds in nanoparticles made of this polymer under optimized conditions. Chitosan is reported to be non-toxic and soft-tissue bio-compatible with soft-tissue. Chitosan is also known to have a special feature of adhering to the mucosal surface and transiently opening the tight junctions between epithelial cells.

Chitosan are both, of large and small molecular weight (MW) and both are commercially available. With good solubility in the physiological pH range, low MW polycataionic Chitosan can be employed for encapsulation of water soluble drugs at pH close to the physiological range. As alternative to encapsulation, aldehyde group of Methylgyloxal can be conjugated to amine group of Chitosan polymer on the nanoparticle surface to prevent release of the drug before nanoparticles reach targeted site.

Encapsulation of water soluble drugs in the mixture of nano and micro sized chitosan nanoparticles for sustained release preparation has been reported by Kauper, Peter et al (US Patent application no 20080254078). Synthesis of chitosan nanoparticles for sustained delivery of proteins and other water-soluble drugs has been described by number of US patents (US patent nos 7,381,716 7,309,500, 7,291,598, 7,265,090 6,649,192,, 20070116772, 20070116771, 20060147539, 20060073210). However, size of these particles exceeds 100nm diameter significantly limiting their extravasations from systemic circulation to tumor tissue through EPR. In the present invention, to reduce the size of the nanoparticles to less than 100nm, we employed Tween80 that form micelles which act as template for the nanoparticles formation over these micelles. The inventive process results into the formation of chitosan nanoparticles of average size generally less than 100nm. Small size of these particles facilitates their extravasation from systemic circulation to tumor tissue through EPR effect.

A US Patent (no. 6, 613, 793) elaborates process of conjugation of Methylglyoxal with amino acids in their formulation that led to improved in vivo stability as well as therapeutic activity of the drug, Methylglyoxal. Hitherto, there is no publication or patent disclosing any suitable carrier like liposomes or nanoparticles for encapsulation of Methylglyoxal and its sustained release allowing its use as a therapeutic agent.

The present disclosure teaches about nano-encapsulation of methylglyoxal in a variety of polymers for oral and injectable administration and their evaluation as a therapeutic agent. It also describes the process for encapsulation of methylglyoxal in the nanoparticles of chitosan, and further develop a therapeutic formulation with conjugated nano- methylglyoxal as the principal ingredient.

In biological systems, ATP is the universal energy currency. Excitable cells and tissues, such as skeletal and cardiac muscles, brain, photoreceptor cells, spermatozoa and electrocytes, all depend on the immediate availability of vast amount of energy that may be used in a pulsed or fluctuating manner. Because adenylate pool, as well as the ATP/ADP ratio, is key regulator influencing many fundamental metabolic processes, these cells have avoided building a large pool of ATP. Instead, large quantities of "metabolically inert" phosphagens are stored in these cells or tissues. In vertebrate species, phospho-creatine is the sole phosphagen and ATP is continuously and efficiently replenished from the large pool of phospho-creatine through the reaction catalyzed by creatine kinase (CK) (EC 2.7.3.2).

ADP + Phospho-creatine ↔ ATP + creatine

In normal physiological condition the creatine/phosphocreatine system is predominantly found in tissues with high and fluctuating energy demand, such as skeletal muscle, heart and brain. Surprisingly it is not present in hepatocytes that have a constitutively high metabolic rate. This is probably due to more or less continuous not fluctuating demand of ATP for this cell.

In rapidly growing cells such as malignant cells, demand for ATP is extremely high, but more or less continuous similar to liver cells but in contrast to excitable cells with fluctuating energy requirement. Because creatine metabolism is intimately connected with ATP requirements, its role in malignant cells is of prime importance.

Because creatine-CK system is possibly intimately related with tumor metabolism through regulation of ATP production and/or modulation the field of creatine-CK system in relation to malignancy had remained under intense investigation. The anticancer effect of creatine and its analogue cyclocreatine had been known for quite some time. Growth rate inhibition of subcutaneously implanted tumors such as rat mammary tumors, sarcoma, human neuroblastoma cells etc in rat or in nude mice was observed when the animal's diet contained creatine and/or cyclocreatine. Cyclocreatine inhibited the proliferation also of the L1236, a Hodgkin-disease derived cell line nearly entirely. Moreover cyclocreatine had a unique mechanism of anticancer activity and is well-tolerated by cancer patients. Augmentation of the anticancer effect of methylglyoxal and ascorbic acid by creatine had been observed in my laboratory.

Recent literature has shown that creatine/CK system is downregulated in many cancerous tissues but did not investigate this downregulation with the progression of malignancy. A systematic study of the status of creatine/CK system with the progression of malignancy was done by Patra et al. In our laboratory, we observed that creatine content progressively decreased in sarcoma tissue of mice with progression of malignancy and the activity of CK reached very low level in the final stage of tumor development in comparison to the Creatine content and activity of CK in normal contralateral muscle of the same animal. We also investigated the status of Creatine and CK in a few post-operative tissue samples from human patients, and similar to the results of animal tissues, the Creatine content and CK activity were much reduced in both human Fibrosarcoma and Gastro-Intestinal Tract Malignancy, compared to healthy control tissues.

Interestingly we observed that treatment of Sarcoma-bearing mice by Methylglyoxal and Ascorbic Acid, both Creatine content and activity of CK were restored significantly with the concomitant decrease of tumor size. Moreover Creatine administration significantly augmented the effect of Methylglyoxal and Ascorbic Acid and thus Creatine has a strong synergistic effect with anticancer activity of Methylglyoxal.

The present invention relates to development of anticancer formulation with creatine as an adjuvant with conjugated Nano- Methylglyoxal as the principal ingredient.

Melatonin is an indole molecule and an endogenous substance released from the pineal gland, one of the seven major human endocrine glands. Recent experimental studies have indicated that the physiological significance of the pineal is that of a central regulator of the immune-neuro-endocrine interactions in relation to either the endogenous homeostasis or universal information, namely the light/dark photoperiod and magnetic fields. In contrast to several other endocrine glands, which secrete hormones that are potentially able to promote cancer cell proliferation the pineal seems to be the only endocrine gland that has a physiologically predominant anticancer role through the circadian release of several potentially antitumor indole and peptidergic hormones, the best known of which is Melatonin. Moreover, several clinical studies had demonstrated that cancer progression is associated with a progressive decline in the pineal function and in melatonin secretion, mainly during the dark period of the day. From this point of view, the first significance of melatonin therapy in advanced cancer patients is that of the classic endocrine replacement treatment of cancer progression-related pineal deficiency.

The other biological mechanisms justifying Melatonin administration in cancer treatment as a possible new natural anticancer agent consist of antiproliferative activity, immunostimulatory, anti-oxidant activity, thrombopoietic action, psychomimetic properties, namely anti-anxiety, antidepressant and anti-asthenic effects.

With these rationales, Melatonin has been applied to treat cancer in both animal and human in our laboratory and has shown significant improvement over the formulations without Melatonin. The significant anticancer effect of Melatonin had also been demonstrated *in vitro.* Ying et al showed that Melatonin inhibited the growth of malignant melanomas *in vitro.* The effects of oral supplementation of Melatonin on growth of Ehrlich Ascites Carcinoma (EAC) cells implanted intra-peritoneally in female mice were studied in our lab. Melatonin at 50 mg/kg body wt. reduced the viability and volume of Ehrlich Ascites Carcinoma cells and increased the survival of the treated mice. It has strong antioxidant properties and anticancer and onco-static action. Melatonin has potential anticancer activity whether given alone or in association with cancer chemotherapy. Jung et al showed that Melatonin has been investigated in advanced solid tumors and brain metastases. In this study, 30 patients with untreatable metastatic solid tumors were treated with daily 30mg oral Melatonin in conjunction with low doses of antitumor cytokines. The lymphocyte proliferation and anticancer immunity of cytokines were found to be significantly increased with Melatonin treatment. In addition Melatonin may have other biological effects, which could be useful in palliative therapy of Cancer. Further, several studies have suggested that combination therapies such as Melatonin and chemotherapeutic agents may increase success by increasing efficacy and decreasing side effects.

The present disclosure relates to the encapsulation and simultaneous chemical Schiffs base conjugation of Methylglyoxal in the nanoparticles of chitosan, an industry adoptable and scalable process for preparing safe Methylglyoxal conjugated Chitosan Nanoparticles and develop a safe and effective Anti-Cancer formulation composed of Methylglyoxal conjugated Nanoparticle, Ascorbic Acid, Creatine and Melatonin.

### OBJECTS OF THE INVENTION

An object of this invention is to propose a nanoparticulated form of chemically conjugated methylglyoxal for inhibition and/or treatment of different types of malignancies with and without metastasis; to treat infections including fungal infections and to use as immune-modulator.

Another object of the invention is to propose a process for preparing nanoparticles of chitosan, crosslinked through glutaraldehyde, and or by other substance, chemically conjugated with methylglyoxal, on the surface of the particle to the maximum extent possible.

Yet another disclosure is a process for the preparation of nanoparticles of chitosan loaded with methylglyoxal, its derivatives or analogs dispersed in aqueous solution, in presence of minimum amount of glutaraldehyde so that entire glutaraldehyde is present in bonded form and no quantifiable amounts of free glutaraldehyde is in the solution.

Further disclosed is a process for the preparation of nanoparticles of chitosan, cross-linked through glutaraldehyde, loaded with methylglyoxal, its derivatives or analogs with polyethyleneglycol chains at the outer surface of the nanoparticle by using suitable micelles of surfactant such as, Tween 80 or polysorbate 80 or other surfactants acting as template for chitosan aggregation.

Another disclosure is to use minimum amount of drug in the dosage form and to target maximum amount of drug to tumors and only negligible amounts to other tissues, which obviates the disadvantages associated with the prior art.

Another disclosure is a process for the easy preparation of nanoparticles of chitosan loaded with methylglyoxal, its derivatives or analogs dispersed in aqueous solution, and formulated an effective anticancer drug composed of methylglyoxal conjugated nanoparticle, melatonin, creatine and ascorbic acid.

Still another disclosure is a nano-drug formulation to inhibit the growth of pathogen cells without adversely affecting healthy cells/tissues.

### BRIEF DESCRIPTION OF THE INVENTION

According to this invention there is provided Sustained Release Formulations containing Methylglyoxal for inhibition and/or treatment of different types of malignancies with and without metastasis; to treat infections including fungal infections and to use as immune-modulator, comprising 0.125-0.5 mg of Methylglyoxal as conjugated to nanoparticles of Chitosan or its derivatives; 0.125-0.5mg of Melatonin, 75-300 mg of Creatine, and 25-100 mg of Ascorbic Acid. In accordance with this invention there is also provided a process for the preparation of nano drug formulation comprising: preparing a homogenons solution of Chitosan; adding Methylglyoxal solution to the said homogenons solution to form a reaction mixture; adding a surfactant to the mixture; subjecting the reaction mixture to the step of stirring/mixing; adding Sodium Sulfate to the mixture under stirring/mixing; adding a cross-linking agent and finally Sodium Metabisulphite to the solution, subjecting the solution to the step of filtration.

### BRIEF DESCRIPTION OF THE ACCOMPANY DRAWINGS

Fig 1 : shows the dynamic light scattering data of the aqueous solution of Nano-Methylglyoxal.
Fig 2: shows the transmission electron micrograph of Nano-Methylglyoxal.
Fig 3: shows the photograph of Ehrlich ascites carcinoma (EAC) cells inoculated mice treated intravenously with modified Nano-Methylglyoxal after 12 days.
Fig 4: shows the sarcoma-180 cells inoculated in mice muscle treated intravenously with Nano-Methylglyoxal and bare Methylglyoxal after 30 days.
Fig 5: shows the histological studies on sarcoma bearing mice treated with bare MG/Nano-MG.
Fig 6: the nano-MG showed enhanced macrophage production and phagocytic activity in the sarcoma bearing mice and it also increased the phagocytic activity of those macrophages in the peritoneal cavity of mice. A part from that superoxide and nitrite production necessary for macrophages for the respiratory burst mechanism is also increased by this treatment.
Fig 7: shows the nano-MG showed increased production of various immunomodulators/cytokines.
Fig 8: shows the histological examination of different organs of mouse to examine the toxicity of Nanomethyl glyoxal.
Figs 9 and 10 show Table I (see below).
Fig 11 shows Table 11 (see below).
Fig 12 shows anti-fungal activity of methylglyoxal.

### DETAILED DESCRIPTION OF THE INVENTION

According to this invention there is provided Novel Sustained Release Formulations containing Methylglyoxal for inhibition and/or treatment of different types of malignancies with and without metastasis; to treat infections including fungal infections and to use as immune-modulator.

The process of this invention comprises dissolving low molecular weight Chitosan in dilute Acetic Acid in the presence of Methylglyoxal, Tween 80 and Sodium Sulphate followed by cross-linking with minimum amount of Glutaraldehyde and finally stabilized by Sodium Metabisulphite when stable cross-linked Nanoparticles of Chitosan is obtained. Nanoparticles of Chitosan so prepared, may be defined as the cross-linked Chitosan Polymer conglomerated to form nanoparticulate on a micellar template of Tween 80.

Methylglyoxal, Ascorbic Acid and Creatine have potent activity for the treatment of cancer. Composition comprising Nano-Methylglyoxal with Ascorbic Acid and Creatine would be useful in the treatment of cancer. In this invention, Melatonin was also recognized as an effective treatment for cancer. It has been clearly evidenced that a composition comprising Nano-Methylglyoxal is effective in treating cancer in combination with Melatonin, Creatine and Ascorbic Acid.

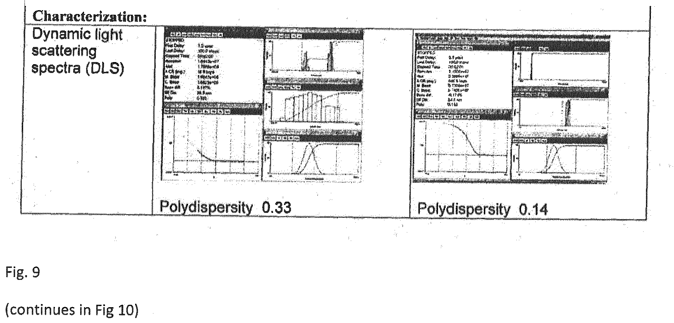

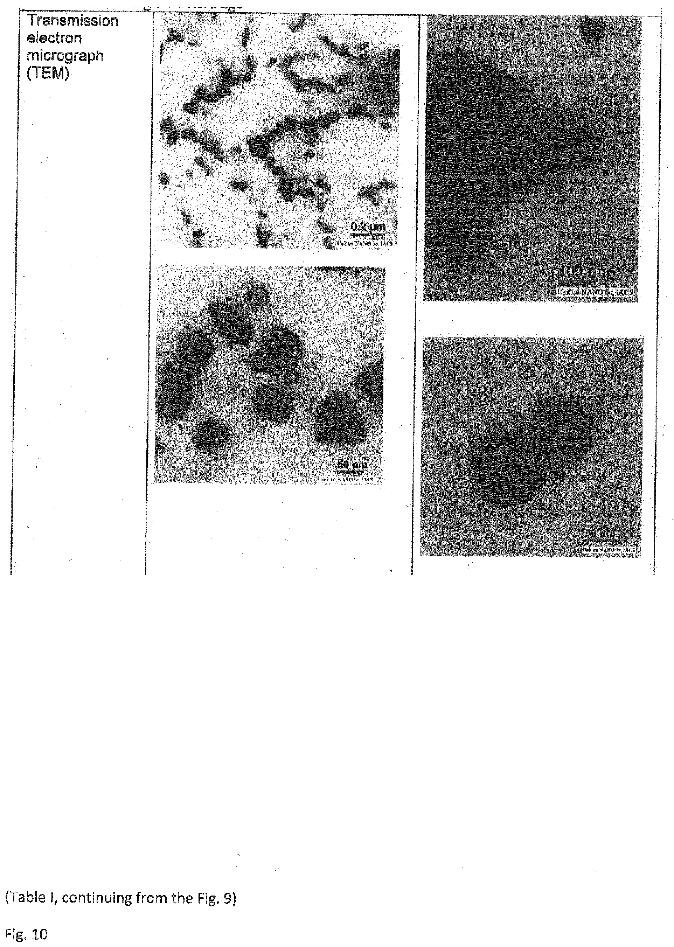

As shown in Table 1 (Figs. 9 and 10), prior art involved dialysis to remove gluteraldehyde from the nano MG formulation, which was not commercially adoptable. Present innovation does not involve dialysis and thus can be used for commercial production. The process time is significantly reduced. The nano particles obtained through the present process are uniformly round in shape, whereas the prior art process gave a mix of irregular shape particles. Yield of MG encapsulated in nano particles is enhanced by 4 times. As compared to the prior art, the present formulation is more effective with fewer doses with less amount of drug and allows tumor regression in shorter durations (Table-2, Table-3).

**Table-1 Process Comparision between modified NanoMG with NanoMG of prior art (reference)**

| | Nano-methylglyoxal of prior art | Modified nanomethylglyoxal |
|---|---|---|
| **Preparation of drug:** | | |
| Total volume of drug prepared | 100ml | 100ml |
| Amount of 40% MG | 1ml | 1ml |
| Chitosan | 150mg | 300mg |
| Glacial acetic acid (17.4M) | 100µl (17.4mM in solution) | 200µl (34.8mM in solution) |
| Tween80 | 1.5ml | 1.0ml |
| Sodium sulphate | 100mg | 80mg |
| 25% Gluteraldehyde | 100 µl (25mg) | 10µl(2.5mg) |
| Dialysis | Twice | No dialysis |
| | 1. against water for 24hrs with two changes followed by | Requisite amount of sodium chloride is added to reach the concentration of NaCl 0.9% to maintain the isotonicity |
| | 2. another 24hrs against normal saline with two changes | |
| Yield (amount of MG anchored in drug measured spectrophotometrically) | 25µg/ml | 10Cµg/ml |
| **Conclusion:-** | | |
| 1) Amount of MG anchored in drug has been increased (by four times). | | |
| 2) The overall procedure of drug preparation is more convenient than the former one. | | |

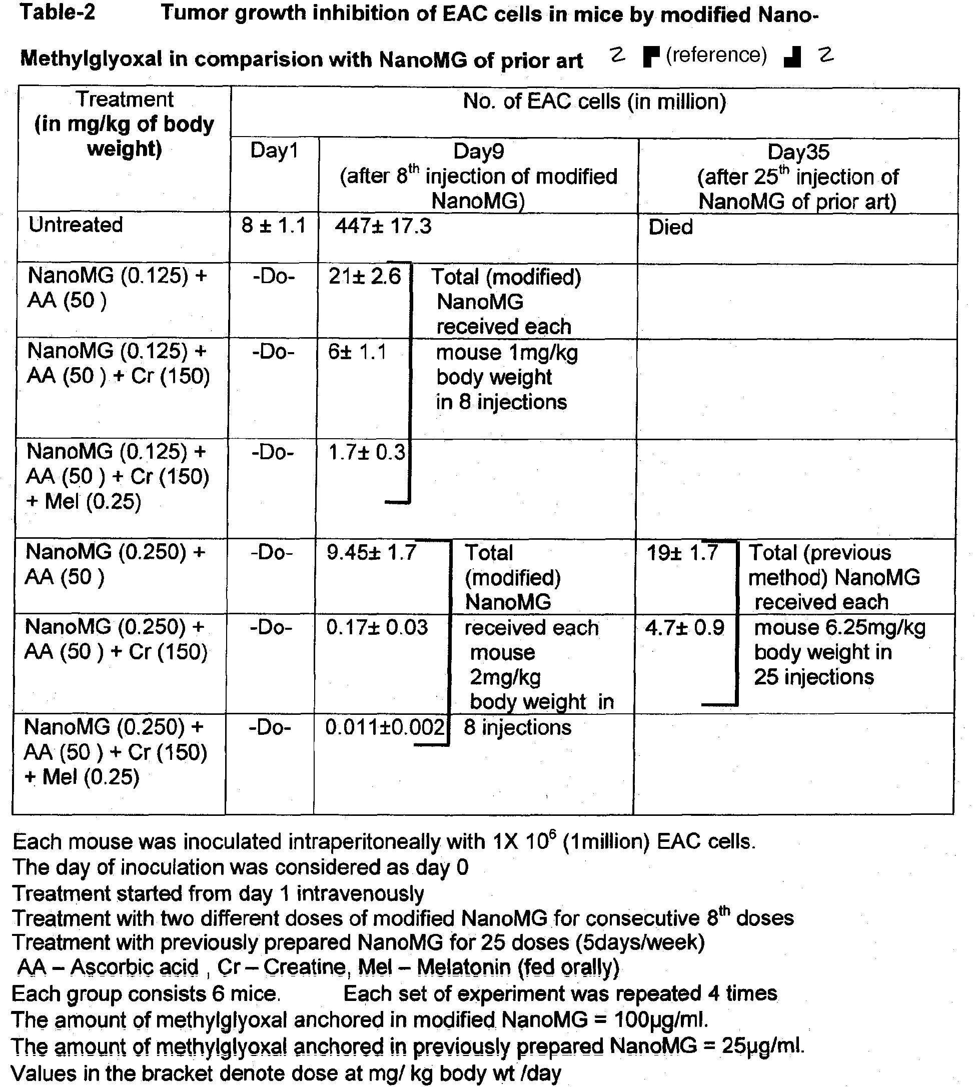

**Table- 3 Comparison of Dose and Effect between modified nanoMG and nanoMG of prior art (reference)**

| In vivo experiment: (in mice model) Each mouse was inoculated intraperitoneally with 1X 10⁶ (1million) EAC cells Treatment started (Intravenously) after 24 hrs of inoculation | | |
|---|---|---|
| Drug & Mode of treatment | NanoMG (of prior art) | NanoMG (modified) |
| | Ascorbic acid & Creatine (fed orally) | Ascorbic acid, Creatine & Melatonin (fed orally) |
| Type of malignant cells | EAC | EAC |
| Dose of drug | 0.25mg/ kg body weight /day | Administered two different doses: |
| | | a) 0.125mg/ kg body weight /day |
| | | b) 0.25mg/ kg body weight /day |
| Total Doses | 25 doses (five days in a week) | 8 consecutive doses for both experiments |
| Total amount of drug administered | 6.25 mg / kg body weight | a) 1 mg / kg body weight |
| | | b) 2 mg / kg body weight |
| Conclusion:- | | |
| 1. Efficacy of modified NanoMG is 5-6 times more and also in shorter durations than the nanoMG of prior art. | | |
| 2. Efficacy is enhanced further when melatonin is introduced in combination with ascorbic acid and creatine for tumor growth inhibition of EAC cells in mice | | |

Biodegradable synthetic polymers (Poly-lactide-co-glycolide: PLGA, Polylactide: PLA, Polycaprolactone: PCL, Eudragit etc.), natural polymers (Alginate-chitosan, solid lipids etc.) and dendrimers have shown promise in encapsulating a diverse range of hydrophobic and hydrophilic molecules of biological interest. These polymers not only help in designing different delivery systems but have flexibility in the route of administration.

The bioavailability and efficacy of anti-cancer drug methylglyoxal was studied/investigated using synthetic polymers (PLGA, PLA, PCL, Eudragit), natural polymers (Alginate-chitosan, solid lipids) and dendrimers. The size of nanoparticles so formed were of <500ηm with a drug loading efficacy of 2.8 - 4.25 µg/mg of nanoparticles (PLGA), 184-705 mg/gm nanoparticles (alginate-chitosan), 10-20mg/gm nanoparticles (SLN).

Methylglyoxal conjugated Chitosan Nanoparticles were prepared by varying concentration of one of their reactants keeping others constant and optimal concentration/ amount of each reactant was determined (table 4). 100ml of 0.1% to 0.4% (optimal 0.3%) low molecular weight Chitosan (mol wt ∼40kD) aqueous solutions in dilute Acetic Acid of 17.5mM to 87.5mM (optimal 35mM) were prepared. To the above homogeneous solutions, 40% w/v Methylglyoxal solution were added varying the amount by 0.5ml to 2.0 ml (optimal 1ml). 0.5ml to 2.0 ml Tween80 (optimal 1ml) were added in the reaction mixtures, followed by addition of 0.2ml to 1ml (optimal 0.4ml) 20% Sodium Sulfate solution. To crosslink, 5µl to 100µl (optimal 10µl) of 25% Gluteraldehyde solution were added to the solutions and the reaction continued for 30 minutes. Finally 0.5ml to 2ml (optimal 1ml) of 10% Sodium Metabisulphite was added.

**Table - 4 Range of reactants and their optimal concentration/ amount, in the production process of Modified NanoMG (Present Formulation)**

| Name of the reactant | Varying concentration/ amount | Optimal concentration /amount |
|---|---|---|
| Acetic acid (Glacial) | 17.5mM - 87.5mM | 35mM |
| Chitosan (mol wt ∼40kD) | 0.1% - 0.4% | 0.3% |
| Methylglyoxal (40% solution) | 0.5ml- 2.0 ml | 1ml |
| Tween 80 | 0.5ml - 2.0 ml | 1ml |
| Sodium sulphate (20% solution) | 0.2ml - 1ml | 0.4ml |
| Gluteraldehyde (25% solution) | 5µl - 100µl | 10µl I |
| Sodium-meta-bisulphite (10% solution) | 0.5ml - 2ml | 1ml |

### Total Volume of the reaction mixture was 100ml

### EXAMPLE- 1

### Preparation of nanomethylglyoxal (NanoMG)

Synthesis of Methylglyoxal conjugated Chitosan Nanoparticles (**Nano-Methylglyoxal):**
A 100ml of 0.3% low molecular weight Chitosan (mol wt ∼40kD) solution in dilute Acetic Acid (35mM) was prepared. To the above homogeneous solution, 1.0 ml of 40% w/v Methylglyoxal solution was added. Tween80 (1.0 ml) was added in the reaction mixture, followed by addition of 0.4ml of 20% Sodium Sulfate solution. To crosslink, 10µl of 25% Gluteraldehyde solution was added to the solution and the reaction continued for 30 minutes. Finally 1ml of 10% Sodium Metabisulphite was added. The formulation in Normal Saline (0.9% NaCl) was filter sterilized, stored at 10-15°C and directly used for further characterization and therapeutic use.

### Characterization of Nano-Methylglyoxal

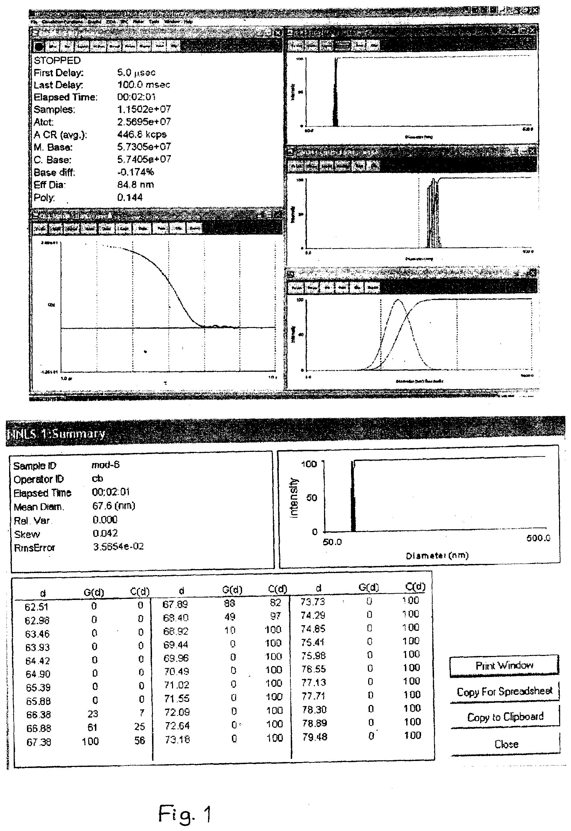

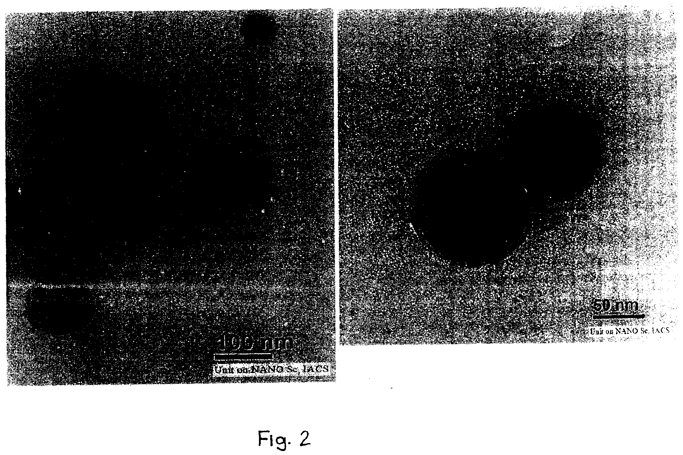

(i) Dynamic light scattering (DLS) spectra of nano-methylglyoxal (Fig.1) showed the polydispersed nature having the particle diameter ranging from 25 to 85 ηm. However, predominantly these particle ranged from 66 to 69 nm.
(ii) Particle size of nano-methylglyoxal measured by Transmission electron micrograph TEM) (Fig.2) was also corroborated with the DLS result.
(iii) Estimation the amount of methylglyoxal anchored in chitosan nanoparticles :
   Methylglyoxal conjugated chitosan nanoparticles solution (0.5ml) was passed through sephadex G-50 column (20X1cm) to eliminate small molecule using normal saline as elutant. The effective portions were collected and estimated for methylglyoxal. The solution was treated with 5M perchloric acid solution so that the methylglyoxal was released from the nanoparticles and it was derivatised with 1,2 diaminobenzene to produce 2-methylquinozaline and was estimated spectrophotometrically by measuring the absorbance at 336nm. The concentration of perchloric acid and that of 1,2 diaminobenzene in reaction mixture were 0.5M and 1.8mM respectively. The amount of methylglyoxal anchored in drug loaded nanoparticles was calculated from a standard curve and is found to be≈ 100µg/ml.

### (iv) In vivo Experiment: with nano-methylglyoxal

The effect of nano-methylglyoxal on the excessive proliferation of cancerous cells in mice was investigated as follows:
a) Mice bearing Ehrlich ascites carcinoma cells (No. of EAC cell inoculated each mouse = 1x 10⁶) were treated intravenously with nano-methylglyoxal in normal saline to observe the effect of cell proliferation. Treatment started after 24 hrs, of inoculation in mice intravenously with three different doses of nanoMG in solution. In first group every day each mouse received 25µl nanoMG solution (containing 2.5µg methylglyoxal), in second group each mouse received 50µl nanoMG solution (containing 5µg methylglyoxal) and in third group each mouse received 100µl nanoMG solution (containing 10µg methylglyoxal). Mice in the negative control group were similarly treated intravenously with normal saline and the positive control groups were treated intravenously with bare methylglyoxal (20mg/kg body weight /day) in normal saline. Each group contains six mice. Each set of experiment was repeated four times. Melatonin (0.25mg/kg body weight /day), creatine (150 mg/kg body weight /day) and Ascorbic acid (50 mg/kg body weight /day) were fed orally to each mouse. Treatment was continued for consecutive 8 days (Table-5, Table-6). Varying the concentrations of melatonin, creatine and ascorbic acid, the change of efficacy of nano-methylglyoxal on tumor regression were also observed (Table-7).

**Table-5 Efficacy of nano-methylglyoxal in comparison to bare methylglyoxal (reference) (in mice model)**

| Drug | Nano-methylglyoxal | Bare-methylglyoxal | None |
|---|---|---|---|
| Mode of treatment | Intravenous | intravenous | intravenous |
| Type of malignant cells | EAC | EAC | EAC |
| No. of cells inoculated on day 0 | 1x10⁶ cells | 1x10⁶ cells | 1x10⁶ cells |
| No. of cells on day | 8 ± 1.1 x10⁶ cells | 8 ± 1.1 x10⁶ cells | 8 ± 1.1 x10⁶ cells |
| Total Doses | 8 consecutive doses starting from day1 | 8 consecutive doses starting from day1 | None |
| Dose of drug | Administered three different doses: | 20mg/ kg body weight /day | None |
| | a) 0.125mg/ kg body weight /day | | |
| | b) 0.25mg/ kg body weight /day | | |
| | c) 0.50mg/ kg body weight /day | | |
| Total amount of drug administered | a) 1 mg / kg body weight | 160mg/ kg body weight | None |
| | b) 2 mg / kg body weight | | |
| | c) 4mg/ kg body weight | | |
| No. of cells on day 9 | 38± 3.28 | 29 ± 2.9 | 447± 17.3 |
| | 13.3± 2.18 | | |
| | 8.9± 1.15 | | |

**Table-6 Efficacy of nano-methylglyoxal in combination of ascorbic acid, creatine and melatonin**

| **Treatment (in mg/kg of body weight)** | **No. of EAC cells (in million)** | | | |
|---|---|---|---|---|
| | **Day 0 (inoculation)** | **Day1 (initiation of treatment)** | **Day9 (after 8 doses of drug)** | |
| Untreated | 1 | 8 ± 1.1 | 447± 17.3 | |
| NanoMG (0.125) | -Do- | -Do | 38± 3.28 | |
| NanoMG (0.125) + AA (50) | -Do- | -Do- | 21± 2.6 | Total NanoMG received each |
| NanoMG (0.125) + AA (50) + Cr (150) | -Do- | -Do- | 6± 1.1 | mouse 1mg/kg body weight in 8 consecutive doses in 8 days |
| NanoMG (0.125) + AA (50) + Cr (150) + Mel (0.25) | -Do- | -Do- | 1.7± 0.3 | |
| NanoMG 0.250 | -Do- | -Do | 13.3± 2.18 | |
| NanoMG (0.250) + AA (50) | -Do- | -Do- | 8.05± 1.07 | Total NanoMG received each |
| NanoMG (0.250) + AA (50) + Cr (150) | -Do- | -Do- | 1.5± 0.18 | mouse 2mg/kg body weight in 8 consecutive doses in 8 days |
| NanoMG (0.250) + AA (50) + Cr (150) + Mel (0.25) | -Do- | -Do- | 0.26±0.02 | |
| NanoMG (0.50) | -Do- | -Do | 8.9± 1.15 | |
| NanoMG (0.50) + AA (50) | -Do- | -Do- | 5.05± 1.07 | Total NanoMG received each |
| NanoMG (0.50) + AA (50) + Cr (150) | -Do- | -Do- | 1.01± 0.08 | mouse 4mg/kg body weight in 8 consecutive doses in 8 days |
| NanoMG (0.50) + AA (50) + Cr 150 + Mel (0.25) | -Do- | -Do- | 0.17±0.02 | |

**Table-7 Efficacy of Nano-methylglyoxal with variation of dose of NanoMG, ascorbic acid, creatine and melatonin**

| | **Treatment (in mg/kg of body weight)** | **No. of EAC cells (in million)** | |
|---|---|---|---|
| | | **Day 1** | **Day 9 (after 8^{th} injection)** |
| | **Untreated** | **8 ± 1.1** | **447± 17.3** |
| Variation of NanoMG concentration | NanoMG (0.125) + AA (50) + Cr (150) + Mel (0.25) | -Do- | 1.7± 0.30 |
| | NanoMG (0.250) + AA (50) + Cr (150) + Mel (0.25) | -Do- | 0.26±0.02 |
| | NanoMG (0.50) + AA (50) + Cr (150) + Mel (0.25) | -Do- | 0.17±0.02 |
| Variation of ascorbic acid concentration | NanoMG (0.250) + AA (25) + Cr (150) + Mel (0.25) | -Do- | 0.49±0.08 |
| | NanoMG (0.250) + AA (50) + Cr (150) + Mel (0.25) | -Do- | 0.26±0.02 |
| | NanoMG (0.250) + AA (100) + Cr (150) + Mel (0.25) | -Do- | 0.22 ±0.03 |
| Variation of creatine concentration | NanoMG (0.250) + AA (50) + Cr (75) + Mel (0.25) | -Do- | 0.37±0.04 |
| | NanoMG (0.250) + AA (50) + Cr (150) + Mel (0.25) | -Do- | 0.26±0.02 |
| | NanoMG (0.250) + AA (50) + Cr (300) + Mel (0.25) | -Do- | 0.21±0.02 |
| Variation of melatonin concentration | NanoMG (0.250) + AA (50) + Cr (150) + Mel (0.125) | -Do- | 0.43±0.09 |
| | NanoMG (0.250) + AA (50) + Cr (150) + Mel (0.25) | -Do- | 0.26±0.02 |
| | NanoMG (0.250) + AA (50) + Cr (150) + Mel (0.50) | -Do- | 0.15±0.02 |

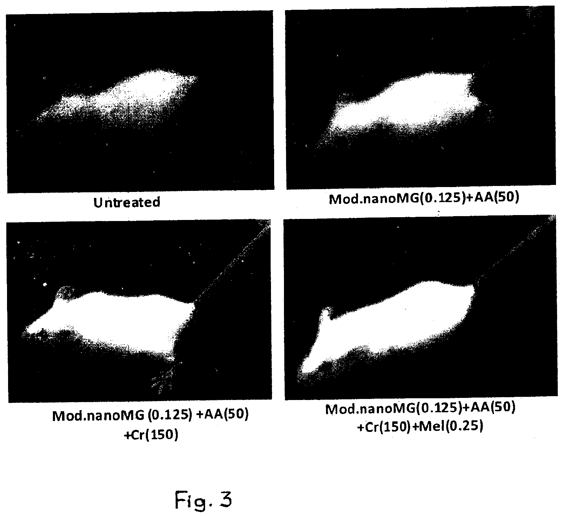

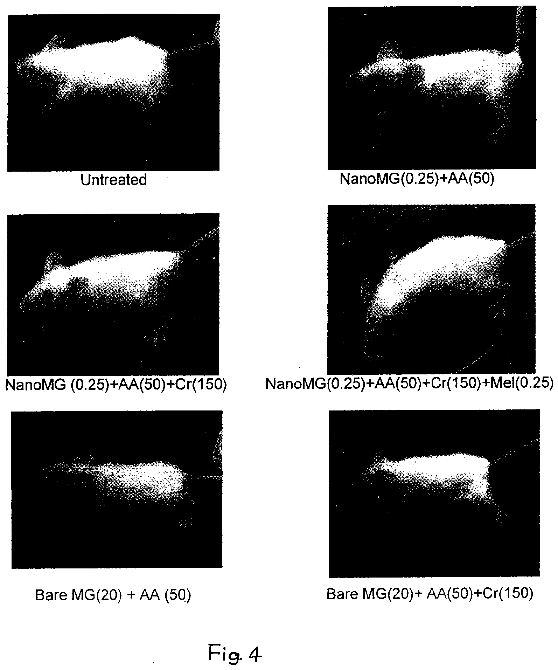

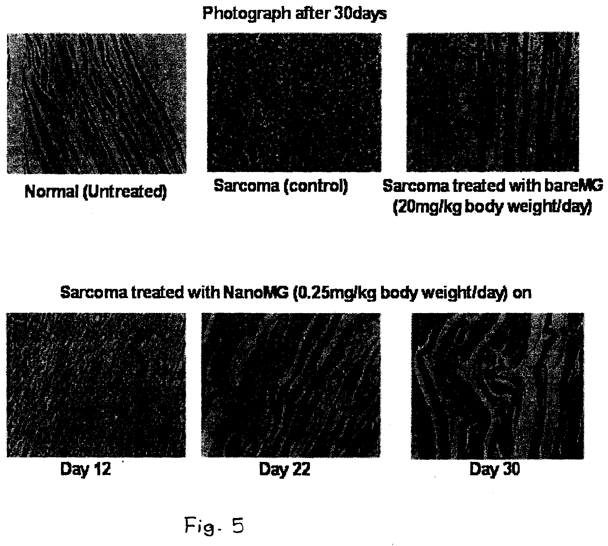

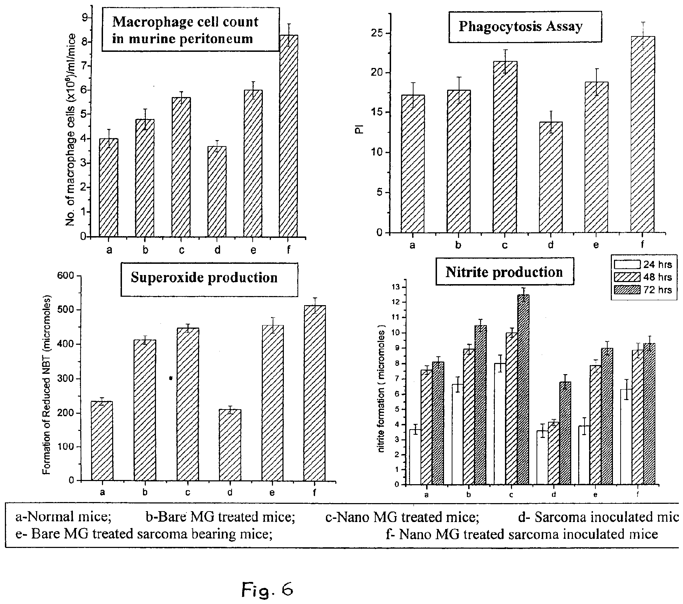

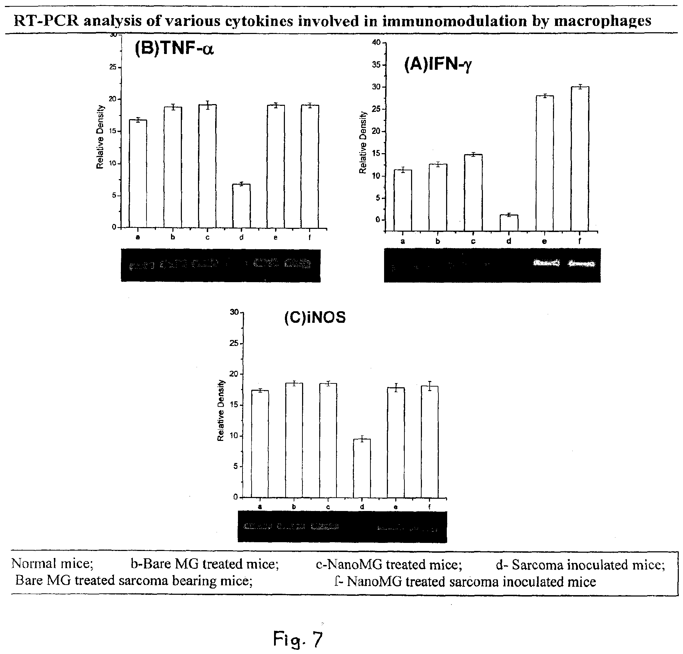

Each mouse was inoculated intraperitoneally with 1X 10⁶ (1million) EAC cells.
The day of inoculation was considered as day 0
Treatment started from day 1 intravenously with NanoMG for consecutive 8^{th} doses AA - Ascorbic acid, Cr - Creatine, Mel - Melatonin (fed orally)
Each group consists 6 mice. Each set of experiment was repeated 4 times The amount of methylglyoxal anchored in NanoMG = 100µg/ml.
Values in the bracket denote dose at mg/ kg body wt /day
Ehrlich ascites carcinoma (EAC) cells developed in the intraperitoneal cavity of mouse were collected in different time intervals. After washing with normal saline the cells were re-suspended in phosphate buffered saline. Cells were counted by the Trypan Blue dye exclusion method using haemocytometer.
Inhibition of proliferation of cancerous (EAC) cells in mice treated intravenously with modified Nano-Methylglyoxal after 12 days was shown in Fig.3.
b) Solid tumor was developed by injecting Sarcoma-180 cells in hind leg of mice. Number of Sarcoma-180 cells inoculated to each mouse was 1x10⁶. The tumor bearing mice were treated with modified nano-methylglyoxal (present formulation) in normal saline through intravenous route to study the tumor growth inhibition by nano-methylglyoxal. Treatment started after 7 days of inoculation in mice intravenously with 50µl nanoMG in solution (containing 5µg methylglyoxal). Mice in the negative control group were similarly treated intravenously with normal saline and the positive control groups were treated intravenously with bare methylglyoxal (20mg/kg body weight /day) in normal saline. Each group contained six mice. Each set of experiment was repeated four times. melatonin (0.25mg/kg body weight /day), creatine (150 mg/kg body weight /day) and Ascorbic acid (50 mg/kg body weight /day) were fed orally to each mouse everyday. Treatment was given with single daily dose for consecutive 12 days. Fig.4 showed the regression of solid tumor in mice model.
c) Solid tumor bearing mice treated with Nano-MG and bareMG as described previously was sacrificed on day 30 and the histological examination of the skeletal muscle was done (Fig. 5). Treatment started after 7 days of inoculation in mice intravenously with 50µl nanoMG in solution (containing 5µg methylglyoxal). Treatment was continued for 20 doses (six doses per week) in 23 days. Melatonin (0.25mg/kg body weight /day), creatine (150 mg/kg body weight /day) and Ascorbic acid (50 mg/kg body weight /day) were fed orally to each mouse.
d) Solid tumor bearing mice treated with nanoMG as described previously were sacrificed for the isolation of macrophages. Briefly, RPMI-1640 was injected in the peritoneal cavity of mouse and peritoneal macrophages were isolated and centrifuged and resuspended in RPMI-1640+10% FBS and used further for immunomodulatory studies. (Results are shown in Fig 6 and Fig 7. Each mouse was inoculated with 0.3x 10⁶ Sarcoma-180 cell (consider as day zero). Treatment started on 3^{rd} day after inoculation in mice with nano MG of 0.25mg/ kg body weight/day and bareMG of 20mg/kg body weight/day. Melatonin (0.25mg/kg body weight /day), creatine (150 mg/kg body weight /day) and Ascorbic acid (50 mg/kg body weight /day) were fed orally to each mouse. Each mouse received 25 doses (5 doses per week). Each experiment was repeated 4 times.

### IMMUNOMODULATORY EFFECT OF NANOMETHYLGLYOXAL AGAINST TUMOR IN MICE:

Methylglyoxal, a normal metabolite is a potent anticancer drug. It can enhance macrophage mediated immunity in murine model by the production of Reactive Oxygen Intermediates (ROIs) and Reactive Nitrogen Intermediates (RNIs) through membrane bound enzyme NADPH-oxidase and iNOS-synthase pathways.

In the present invention, with an objective to enhance the efficacy of this compound by increasing its retention time in body as well as decreasing the dose significantly, a polymer conjugated formulation of the same compound has been developed. In the present invention, effect of modified nanomethylGlyoxal, against tumor in mice, have been studied. Briefly, Swiss albino female mice aged 4-6 weeks weighing 18-20 g were taken. Each group contained four mice. *Tumors were developed in the left hind leg of a mouse by intra-muscular injection of sarcoma-180 cells (approximately 2x10⁶ cells in each mouse).* Each set of experiment was repeated 4 times. Drug was administered to the mice intravenously, five days a week. Polymer conjugated methylglyoxal (Nano-MG) was administered intravenously at an amount of 0.4 mg/kg body weight/ day. One group received bare methylglyoxal (20mg/kg body weight/day). Drug was administered after 72 hours of inoculation of Sarcoma-180 tumour in the treated group. Treatment was continued for four weeks. Results are shown in Table 8, Table 9 and Table10.

**Table-8 Measurement of tumor volume:**

| Days | **Sarcoma-180** Volume of tumour (c.c.) | **Sarcoma-180 + Bare-MG** Volume of tumour (c.c.) | **Sarcoma-180 + Nano-MG** Volume of tumour (c.c.) |
|---|---|---|---|
| 12 | 0.96±0.057 | 1±0.1 | 0.80±0.09 |
| 16 | 2.47±0.74 | 1.4±0.05 | 0.83±0.28 |
| 21 | 5.23±0.90 | 2±0.15 | 0.52±0.10 |
| 26 | 6.97±0.85 | 2±0.17 | 0.44±0.06 |
| 31 | 8.15±0.96 | 2.73±0.27 | 0.45±0.06 |

**Table-9 Peritoneal macrophage Count and Superoxide anion generation**

| **Treatment Batch** | **Peritoneal macrophage Count (X10⁶)** | **Superoxide anion generation Production of reduced NBT (micromoles)** |
|---|---|---|
| **Normal mice** | 9.48 ± 1.28 | 242 ± 2.82 |
| **Bare MG treated normal mice** | 10.88 ± 1.15 | 243 ± 2.82 |
| **Polymer conjugated-MG Treated normal mice** | 12.3 ± 0.42 | 252.5 ± 3.53 |
| **Sarcoma inoculated mice** | 4.46 ± 0.95 | 118 ± 4.24 |
| **Bare MG treated sarcoma tumour bearing mice** | 12.91 ± 1.46 | 317.5 ± 10.60 |
| **Polymer conjugated-MG treated sarcoma tumour bearing mice** | 16.21 ± 1.09 | 378.5 ± 37.47 |

**Table-10 Conclusion of Immunomodulatory Studies:**

| Experiments | Results | Significance |
|---|---|---|
| Measurement of tumor volume | Almost six fold less Tumor volume in nano MG batch than untrested .where as Only two fold reduction in Tumour in bare-MG batch. | Nano-MG is antitumoricidal with respect to bare-MG |
| Isolation& counting of peritoneal macrophages | Macrophage count increased four fold in Nano-MG treated mice, only two fold in bare-MG treated mice with respect to untreated tumour bearing batch | Nano-MG has much more macrophage enhancing property with respect to bare-MG |
| Superoxide anion generation: | Enhanced two times in Nano-methylglyoxal treated tumor bearing animals and 1.5 times in bare-MG group than in the tumour bearing untreated group. | Superoxide radical is one of the major reactive oxygen intermediates found to efficiently scavenge pathogens and debris. |

Very significantly, the present modified polymer conjugated formulation of methylglyoxal showed its immunomodulatory effect, by stimulating the same peritoneal macrophages but in a faster and efficient manner at a significantly low dose in comparison with the reference bare methylglyoxal.

### ANTIBACTERIAL ACTIVITY OF CHITOSAN CONJUGATED METHYLGLYOXAL:

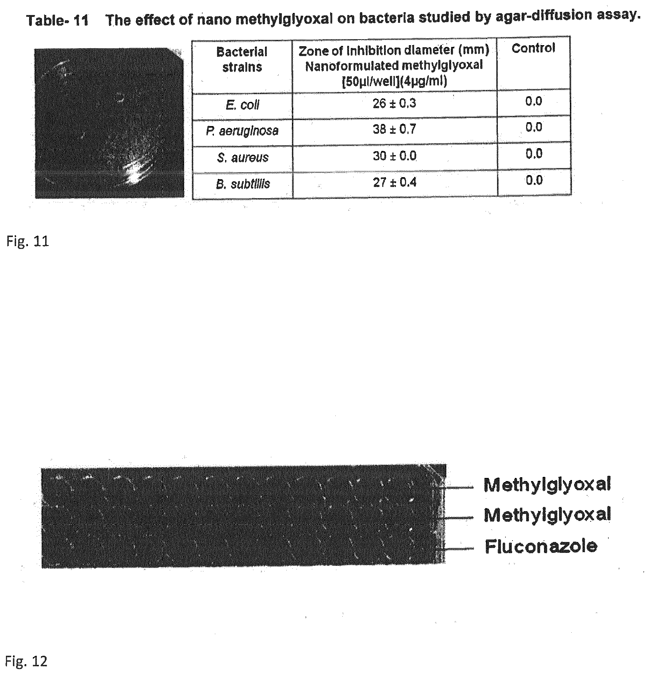

Nanoformulation of methylglyoxal had significant bactericidal activity against multi-drug resistant entero-pathogens including Gram-negative (Pseudomonas aeruginosa, Escherichia coli) and Gram-positive bacteria (Staphylococcus aureus, Bacillus subtilis). Nano methylglyoxal also demonstrated significant antimicrobial activity towards resistant bacteria without having toxicity on human erythrocytes (Table 11, Fig. 11).

### ANTIFUNGAL ACTIVITY OF METHYLGLYOXAL:

**Test Method:** Micro-broth dilution assay according to the NCCLS protocol
**Fungal Pathogens Used:** *Aspergillus niger* and *Candida albicans*

The test was conducted to check Minimum Inhibitory Concentration (MIC)* of the drug towards fungal pathogen. Control drug used in the experiments was Fluconazole.

### Result:

MIC of the drug for *A. niger* - **2.5mg/ml**
MIC of the drug for *C. albicans* - **1.25mg/ml**

^{*}MIC is the minimum concentration of the drug required to inhibit the growth of the micro organism

### TOXICITY STUDY OF NANO-METHYLGLYOXAL ON MICE MODEL:

### Acute toxicity study

For test with mice, the animals were divided in groups each containing 6 animals either male or female weighing 18-20 gms. All the animals received nano-methylglyoxal intravenously in single dose (40µg/mouse/day) of 0.2ml through tail vein. Nano-methylglyoxal solution was passed through a membrane filter of 0.2-micron pore size. The control group received only normal saline. Melatonin, creatine and Ascorbic acid were fed orally.

The maximum dose of nano-methylglyoxal for each mouse was, for intravenous 40µg. All the animals were observed up to 90 days. No death was observed. All the animals remained healthy, no weight loss and behavioral change were observed. No external toxic symptoms were noted in animals in general appearance and in respect of skin and hair texture and in behavioral pattern in respect of food and water intake and activity. No other abnormalities were found.

### Long-term (chronic) toxicity study

Mode of treatment and preparation of nano-methylglyoxal in normal saline was identical to acute toxicity study. All the mice received nano-methylglyoxal 0.2 ml per mice in two divided doses per day (40µg/mouse/day) for a total period of 30 doses in 6 weeks (five days a week due to swelling of tail and adjoining areas). In the entire study, control group received normal saline in similar manner.

Like acute toxicity study all the animals were observed up to 90 days after completion of the treatment and were found to remain healthy. Similar to acute toxicity study, no death and toxic effect (physical and behavioral) were observed during the observation period.

The results of the biochemical tests and some hematological parameter estimated from whole blood/ sera of the nano-methylglyoxal treated mice showed no significant change from untreated normal mice

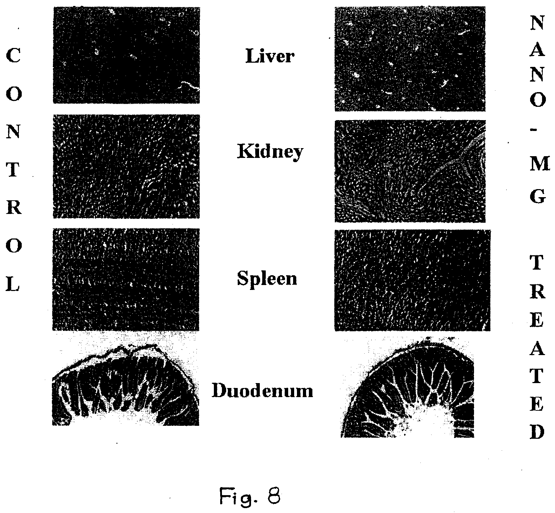

Mortality, general physical and behavioral conditions and changes of body weight if any were observed. Besides observing these parameters, hematological and biochemical tests were also performed in blood samples. Histological studies were done (results in Fig. 8) with several organs of mice subjected to nano-methylglyoxal treatment and compared with that of the untreated animals.

## Claims

1. A sustained release formulation containing methylglyoxal for use in inhibition and/or treatment of malignancies with and without metastasis; for use in treating infections including fungal infections and as immune-modulator medicament, comprising of following constituents:
0.125-0.5 mg of methylglyoxal encapsulated by or conjugated to nanoparticles of bio-degradable synthetic polymers or natural polymers;
0.125-0.5 mg of melatonin;
75-300 mg of creatine; and
25-100 mg of ascorbic acid,
wherein all doses are per kg of body weight.

2. The composition for use in therapy according to claim 1, wherein the natural polymer is chitosan.

3. The composition for use in therapy according to claim 2, wherein the chitosan:
methylglyoxal ratio ranges between 1:0.5 to 1:2.

4. The composition for use in therapy according to claim 2, wherein the dose of methylglyoxal in chitosan nanoparticles is 0.25mg per kg body weight.

5. The composition for use in therapy according to claim 1, wherein the dose of melatonin is 0.25mg per kg body weight, creatine is 150mg per kg body weight, and ascorbic acid is 50mg per kg body weight.

6. A process for the preparation of a nanoparticle drug formulation comprising:
preparing a homogenous solution of chitosan;
adding methylglyoxal solution to the homogenous solution to form a reaction mixture;
adding a surfactant to the reaction mixture;
subjecting the reaction mixture and surfactant to the step of stirring/mixing;
adding sodium sulfate to the surfactant and reaction mixture under stirring/mixing;
adding a crosslinking agent and finally sodium metabisulphite to the solution, subjecting the solution to the step of filtration.

7. The process as claimed in claim 6, wherein the solution
of chitosan is made in dilute acetic acid having concentration in the range of 17.5 mM-87.5 mM.

8. The process as claimed in claim 6, wherein a 40% w/v methylglyoxal solution is added in the concentration range of 0.5 ml - 2.0 ml/100 ml of the total reaction mixture.

9. The process as claimed in claim 6, wherein the surfactant is polysorbate 80 and is added in the range of 0.5 ml - 2.0ml/100 ml of reaction mixture.

10. The process as claimed in claim 6, wherein the carbonyl group of the methylglyoxal is conjugated with an amine group of the chitosan to impart long duration sustained release characteristics.

## Patentansprüche

1. Eine Formulierung mit anhaltender Freisetzung, die Methylglyoxal beinhaltet, zur Verwendung bei der Hemmung und/oder Behandlung von Malignomen mit und ohne Metastasen; zur Verwendung bei der Behandlung von Infektionen, einschließlich Pilzinfektionen, und als Immunmodulator-Medikament, mit folgenden Bestandteilen:
0,125-0,5 mg Methylglyoxal, das durch Nanopartikel biologisch abbaubarer synthetischer Polymere oder natürlicher Polymere verkapselt oder zu diesen konjugiert ist;
0,125-0,5 mg Melatonin;
75-300 mg Creatin; und
25-100 mg Ascorbinsäure,
wobei alle Dosen pro kg Körpergewicht gelten.

2. Die Zusammensetzung zur Verwendung bei einer Therapie gemäß Anspruch 1, bei der das natürliche Polymer Chitosan ist.

3. Die Zusammensetzung zur Verwendung bei einer Therapie gemäß Anspruch 2, bei der das Verhältnis von Chitosan: Methylglyoxal zwischen 1:0,5 und 1:2 variiert.

4. Die Zusammensetzung zur Verwendung bei einer Therapie gemäß Anspruch 2, bei der die Dosis von Methylglyoxal in Chitosan-Nanopartikeln 0,25 mg pro kg Körpergewicht beträgt.

5. Die Zusammensetzung zur Verwendung bei einer Therapie gemäß Anspruch 1, bei der die Dosis von Melatonin 0,25 mg pro kg Körpergewicht beträgt, von Creatin 150 mg pro kg Körpergewicht beträgt und von Ascorbinsäure 50 mg pro kg Körpergewicht beträgt.

6. Ein Verfahren für die Herstellung einer Nanopartikel-Arzneiformulierung, das folgende Schritte aufweist:
Herstellen einer homogenen Chitosan-Lösung;
Zugeben einer Methylglyoxal-Lösung zu der homogenen Lösung, um eine Reaktionsmischung zu bilden;
Zugeben eines oberflächenaktiven Mittels zu der Reaktionsmischung;
Aussetzen der Reaktionsmischung und des oberflächenaktiven Mittels gegenüber dem Schritt eines Rührens/Mischens;
Zugeben von Natriumsulfat zu dem oberflächenaktiven Mittel und der Reaktionsmischung unter Rühren/Mischen;
Zugeben eines Vernetzungsmittels und schließlich von Natriummetabisulphit zu der Lösung,
Aussetzen der Lösung gegenüber dem Schritt einer Filtration.

7. Das Verfahren gemäß Anspruch 6, bei dem die Chitosan-Lösung in verdünnter Essigsäure mit einer Konzentration in dem Bereich von 17,5 mM-87,5 mM hergestellt wird.

8. Das Verfahren gemäß Anspruch 6, bei dem eine Methylglyoxal-Lösung mit 40% Gew./Vol. in dem Konzentrationsbereich von 0,5 ml - 2,0 ml/100 ml der Gesamtreaktionsmischung zugegeben wird.

9. Das Verfahren gemäß Anspruch 6, bei dem das oberflächenaktive Mittel Polysorbat 80 ist und in dem Bereich von 0,5 ml - 2,0 ml/100 ml der Reaktionsmischung zugegeben wird.

10. Das Verfahren gemäß Anspruch 6, bei dem die Carbonyl-Gruppe des Methylglyoxals zur Verleihung lang anhaltender Freisetzungscharakteristika mit einer Amingruppe des Chitosans konjugiert wird.

## Revendications

1. Formulation à libération prolongée contenant du méthylglyoxal destinée à être utilisée dans l'inhibition et/ou le traitement de tumeurs malignes avec et sans métastases; destinée à être utilisée dans le traitement d'infections, y compris les infections fongiques, et comme médicament modulateur immunitaire, comprenant les constituants suivants:
de 0,125 à 0,5 mg de méthylglyoxal encapsulé par ou conjugué avec des nanoparticules de polymères synthétiques biodégradables ou de polymères naturels;
de 0,125 à 0,5 mg de mélatonine;
de 75 à 300 mg de créatine; et
de 25 à 100 mg d'acide ascorbique,
dans laquelle toutes les doses sont exprimées par kg de poids corporel.

2. Composition destinée à être utilisée dans une thérapie selon la revendication 1, dans laquelle le polymère naturel est le chitosan.

3. Composition destinée à être utilisée dans une thérapie selon la revendication 2, dans laquelle le rapport chitosan:méthylglyoxal est compris entre 1:0,5 et 1:2.

4. Composition destinée à être utilisée dans une thérapie selon la revendication 2, dans laquelle la dose de méthylglyoxal dans les nanoparticules de chitosan est de 0,25 mg par kg de poids corporel.

5. Composition destinée à être utilisée dans une thérapie selon la revendication 1, dans laquelle la dose de mélatonine est de 0,25 mg par kg de poids corporel, celle de créatine est de 150 mg par kg de poids corporel et celle d'acide ascorbique est de 50 mg par kg de poids corporel.

6. Procédé de préparation d'une formulation de médicament à base de nanoparticules comprenant le fait de:
préparer une solution homogène de chitosan;
ajouter une solution de méthylglyoxal à la solution homogène pour former un mélange de réaction;
ajouter un tensioactif au mélange de réaction;
soumettre le mélange de réaction et le tensioactif à l'étape d'agitation/de mélange;
ajouter du sulfate de sodium au tensioactif et au mélange de réaction tout en agitant/mélangeant;
ajouter un agent de réticulation et finalement du métabisulfite de sodium à la solution;
soumettre la solution à l'étape de filtration.

7. Procédé selon la revendication 6, dans lequel la solution de chitosan est préparée dans de l'acide acétique dilué ayant une concentration dans la plage de 17,5 mM à 87,5 mM.

8. Procédé selon la revendication 6, dans lequel une solution de méthylglyoxal à 40% p/v est ajoutée dans la plage de concentrations comprise entre 0,5 ml et 2,0 ml/100 ml du mélange de réaction total.

9. Procédé selon la revendication 6, dans lequel le tensioactif est du polysorbate 80 et est ajouté dans la plage de 0,5 ml à 2,0 ml/100 ml de mélange de réaction.

10. Procédé selon la revendication 6, dans lequel le groupe carbonyle du méthylglyoxal est conjugué avec un groupe amine du chitosan pour conférer des caractéristiques de libération prolongée de longue durée.
